# FASCICULE DE BREVET EUROPEEN

(11) **EP 1 091 975 B1**
(45) Date de publication et mention de la délivrance du brevet: **14.12.2005**
(21) Numéro de dépôt: 99926684.4
(22) Date de dépôt: 30.06.1999
(51) Int. Cl.: C07K 7/64, A61K 38/13

(54) **NOUVELLE CYCLOSPORINE AYANT UN PROFIL D'ACTIVITE AMELIORE**
NEUES CYCLOSPORIN MIT VERBESSERTER WIRKUNG
NOVEL CYCLOSPORIN WITH IMPROVED ACTIVITY PROFILE

(30) Priorité: 01.07.1998 CH 140598
(43) Date de publication de la demande: 18.04.2001
(73) Titulaire: DEBIOPHARM S.A., 1000 Lausanne 9 (CH)
(72) Inventeur: WENGER, Roland, M., CH-4125 Riehen (CH); MUTTER, Manfred, CH-1028 Préverenges (CH); RUCKLE, Thomas, Université de Lausanne, CH-1015 Lausanne (CH)
(74) Mandataire: Nargolwalla, Cyra
(86) Numéro de dépôt international: PCT/IB1999/001232
(87) Numéro de publication internationale: WO 2000/001715

(56) Documents cités:
- EP-A- 0 484 281
- WO-A-97/04005
- PAPAGEORGIOU C ET AL: "ANTI HIV-1 ACTIVITY OF A HYDROPHILIC CYCLOSPORIN DERIVATIVE WITH IMPROVED BINDING AFFINITY TO CYCLOPHILIN A" BIOORGANIC & MEDICINAL CHEMISTRY LETTERS, vol. 6, no. 1, 1996, pages 23-26, 497, XP000615812

## Description

La présente invention traite d'une nouvelle cyclosporine (Cs) de son utilisation pharmaceutique ainsi que d'une composition pharmaceutique la comprenant.

Les Cs sont une classe de undécapeptides cycliques, poly-N-méthylés, possédant plusieurs activités pharmacologiques, en particulier ce sont des agents immunosuppresseurs, anti-inflammatoires, anti-parasitiques, suppresseurs de résistance aux drogues (anti-MDR) et anti-virales. La première cyclosporine isolée à partir d'une culture de champignon est la cyclosporine A, que l'on trouve à l'état naturel et qui est représentée par la formule suivante:

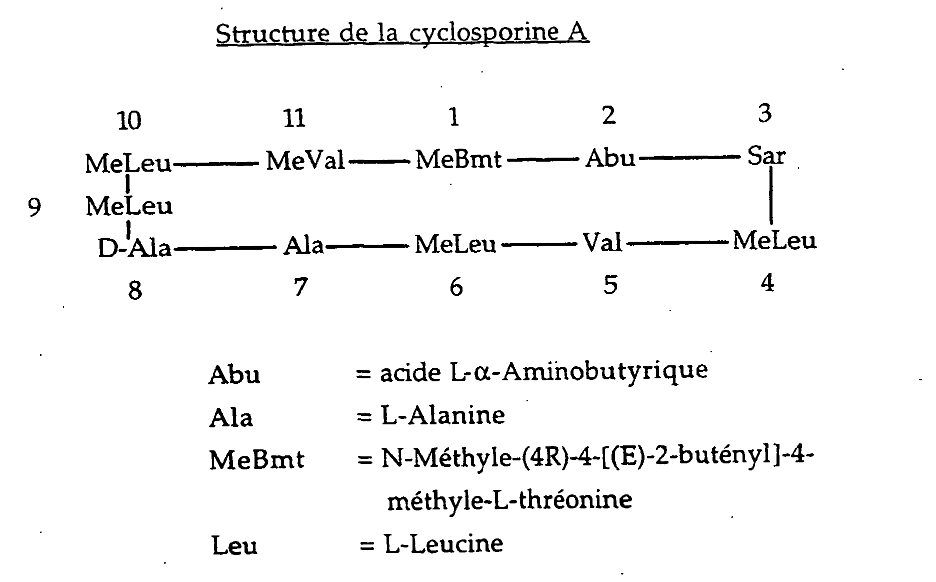

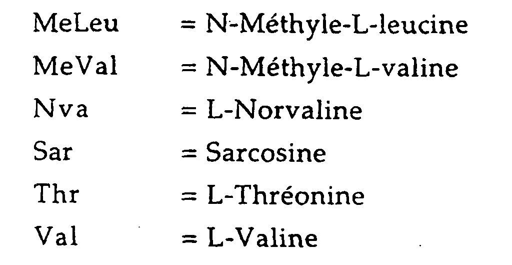

Les acides aminés décrits suivant leur abréviation conventionnelle sont de configuration L à moins qu'il en soit spécifié autrement.

Depuis que cette première cyclosporine a été découverte un grand nombre d'autres variétés ont été isolées et identifiées de même que des variétés non naturelles obtenues par des méthodes synthétiques ou semi-synthétiques, ou même par l'application de techniques de culture modifiées. La production de la cyclosporine A est décrite par [Kobel et al. European Journal of applied Microbiology and biotechnology 14,237-240 (1982)]. On décrit aussi la fabrication de cyclosporines artificielles produites par méthode purement synthétique développée par R. Wenger - voir Traber et al.1, Traber et al. 2 et Kobel et al., US 4,108,985; 4,210,581; 4,220,641; 4,288,431; 4,554,351 et 4,396,542; EP 34 567 et 54 782; WO 86/02080; Wenger 1, Transpl. Proc.15, Suppl. 1:2230 (1983); Wenger 2, Angew. chem. Int. Ed., 24,77 (1985); et Wenger 3, Progress in the chemistry of organic Natural Products 50,123 (1986).

La cyclosporine A (CsA), isolée il y a 20 ans à partir du *Tolypocladuim inflatum* possède une forte activité immunosuppressive. Elle a révolutionné la transplantation d'organes et est couramment utilisée dans le traitement de maladies auto-immunes. Pour une vision récente de l'utilisation de la CsA et ses mécanismes d'action voir Wenger et al: Cyclosporine Chemistry, Structure-activity relationships and Mode of action, Progress in Clinical Biochemistry and Medecine, Vol. 2, 176 (1986).

L'effet thérapeutique de la CsA résulte principalement en la suppression sélective de l'activation des lymphocytes T. Cette activité immunosuppressive s'explique par le fait que la CsA se lie à un récepteur protéique intracellulaire, la cyclophiline A (CyP) formant un complexe CyP-CsA qui va interagir avec la calcineurine (CaN) et inhiber ainsi son activité phosphatase. Ainsi la transcription de familles de gènes à activation précoce sera bloquée (cf:O'Keefe, S.J; Tamura, J; *Nature* 1992, 357, 692-694).

Le sujet de la présente invention est la production d'une nouvelle cyclosporine à forte activité inhibitrice du HIV-1 (virus de l'immunodéficience humaine), n'ayant pas l'activité immunosuppresive de la CsA.

La mode d'infection de HIV de type 1 est unique parmi les rétrovirus car il nécessite l'incorporation spécifique dans son virion de la protéine cellulaire CyP qui va interagir avec la polyprotéine Gag (cf: Eltaly Kara Franke, Bi-Xing Chen Journal of virology, sept. 1995. Vol 69, N°9). Il est connu que la CyP se lie à la CsA et la CaN en un complexe ternaire. Cependant la fonction native de la CyP est de catalyser l'isomérisation des liaisons peptidyl-prolyl, une étape limitante et importante dans le processus permettant aux protéines d'acquérir une structure tridimensionnelle définitive. La CyP protège également les cellules contre les chocs thermiques ou sert de protéine chaperonne. Au contraire de la CsA, le produit du gène Gag du HIV-1 interdit la formation d'un complexe ternaire avec la CyP et la CaN. En fait; le virus HIV a besoin de la Cyp pour se lier avec le produit du gène Gag de manière à constituer ses propres virions (Cf: Franke, E.K;1994 *Nature* 372:359-362). En présence de CsA il y a compétition directe avec la polyprotéine issue du gène Gag de HIV-1 pour se lier à la CyP. Cette CsA va agir à deux niveaux sur la réplication du cycle viral. Tout d'abord au niveau de la translocation vers le noyau du complexe préintégré puis dans la production de particules virales infectieuses.

Le brevet US 4,814,323 décrit déjà une activité anti-HIV de la CsA, cependant celle-ci présente aussi une forte activité immunosuppressive qui n'est pas souhaitée pour le traitement des patients infectés par le virus HIV. Récemment un autre type de cyclosporine a été développé , il s'agit de dérivés en position 4 tels que MeIle⁴ Cs, MeVal⁴ Cs, ou (4-OH) MeLeu⁴-Cs pour ne citer que les substances les plus anti-HIV et les moins immunosuppressives. Le dérivé [(4-OH) MeLeu⁴-Cs] est synthétisé par oxydation de la cycloporine A à l'aide d'un microorganisme. Un autre brevet WO 97/04005 emploie la méthode de préparation du brevet EP 484 281 ainsi que et la méthode développée par Seebach EP 194972 afin de produire des dérivés en position 3, tel que par exemple la (D)-MeSer³-(4-OH)MeLeu⁴-cyclosporine. Cette substance possède une meilleures affinité à la CyP, mais ne possède plus qu'une activité anti-HIV limitée par rapport au dérivé de référence MeIle⁴-CS (NIM 811). Le caractère plus hydrophile de cette substance empêche sa pénétration dans les cellules et dans l'organisme. Ceci se reflète directement sur l'activité anti-HIV réduite de cette substance (cf: Christos Papageorgiou, J.J. Sanglier et René Traber - *Bioorganic & Medicinal Chimistry Letters,* Vol 6, N°1, pp 23-26, 1996).

Les substances décrites dans cette invention présentent le double avantage de conserver la même affinité à la Cyp que celle observée pour la [(4 - OH) Meleu⁴]-Cs ou la cyclosporine A tout en ayant une activité anti-HIV identique voire supérieure à celle des dérivés de références (MeVal⁴-Cs ou MeIle⁴-Cs) et nettement supérieure à l'activité anti-HIV de la cyclosporine A ou de la (4 - OH) MeLeu⁴-CS. Le sujet de l'invention est de fournir une nouvelle cyclosporine, n'ayant pas l'activité immunosuppressive de la CsA et possédant un profil d'activité amélioré. Cette nouvelle famille de Cs est caractérisée par la formule (I): 
X—U—Y—Z—Val—MeLeu—Ala—(D) Ala—MeLeu—MeLeu—MeVal
1 2 3 4 5. 6 7 8 9 10 11
(I)
 dans laquelle:
X est -MeBmt ou 6,7 dihydro-MeBmt-
U est -Abu, Nva, Val, Thr
Y est Sar ou (D)-MeSer ou (D)-MeAla ou(D)-MeSer (OAcyl)
Z est (N-R)aa où aa={Val, Ile, Thr, Phe, Tyr, Thr (OAc), Thr (OG₁),
Phe (G₂), PheCH₂(G₃), Tyr (OG₃)} avec R={alkyl > CH₃, et alkyl < -C₁₀H₂₁};
G₁={Phényle-COOH, Phényle-COOMe, Phényle-COOEt};
G₂={CH₂COOH, CH₂COOMe, CH₂COOEt CH₂PO(OMe)₂, CH₂PO(OH)₂}
G₃={PO(OH)₂, PO(OCH₂CH=CH₂)₂, CH₂COOH, CH₂COOMe, CH₂COOEt}

Ainsi en remplaçant le groupement MeLeu naturel en position 4 par un groupement N-(alkyl) aa (où alkyl > CH₃), et alkyl <-C₁₀H₂₁ on améliore l'activité anti-HIV 1 de ce dérivé.

Dans un mode de réalisation, Z est (N-R) val.

La nouvelle molécule de cyclosporine ainsi obtenue offre l'avantage inattendu et surprenant de présenter une bien meilleure stabilité à la métabolisation que toutes les autres cyclosporines connues jusqu'à présent.

Cette nouvelle molécule résiste beaucoup mieux aux phénomènes d'oxydation et de dégradation qui ont lieu dans la cellule. De ce fait, la durée de vie "in vivo" de cette nouvelle N-alkyl aa Cs se trouve particulièrement prolongée.

De plus, cette nouvelle N-alkyl aa⁴ cyclosporine possède une affinité élevée pour la CyP et présente une activité anti-HIV égale ou supérieure aux meilleures cyclosporines existantes.

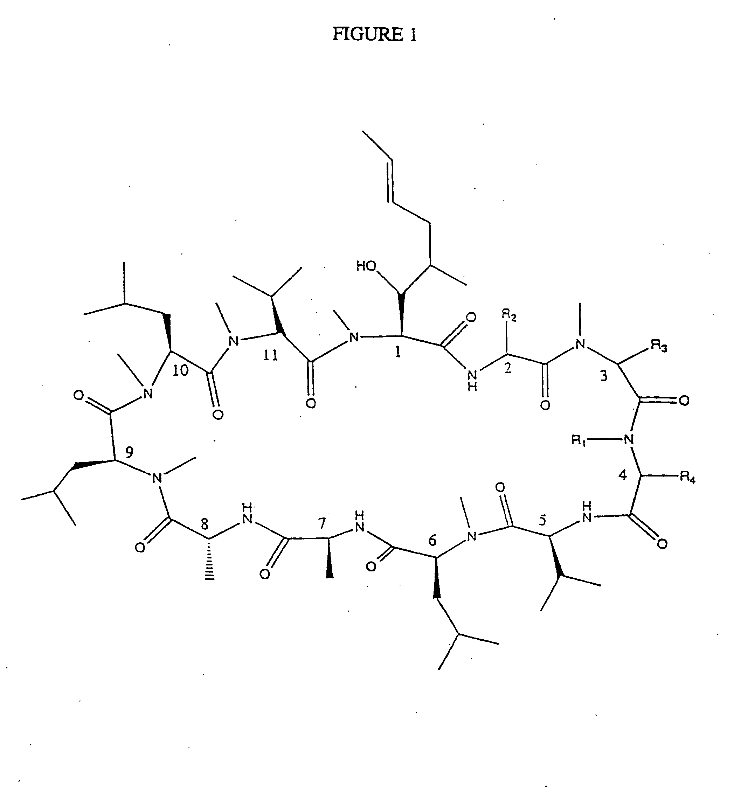

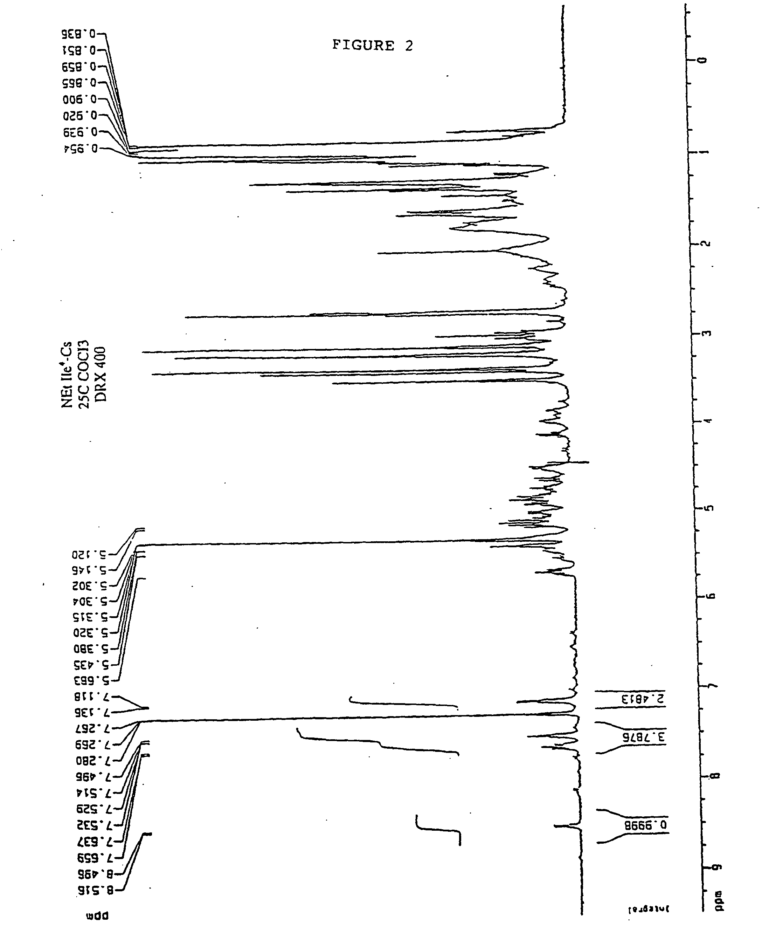

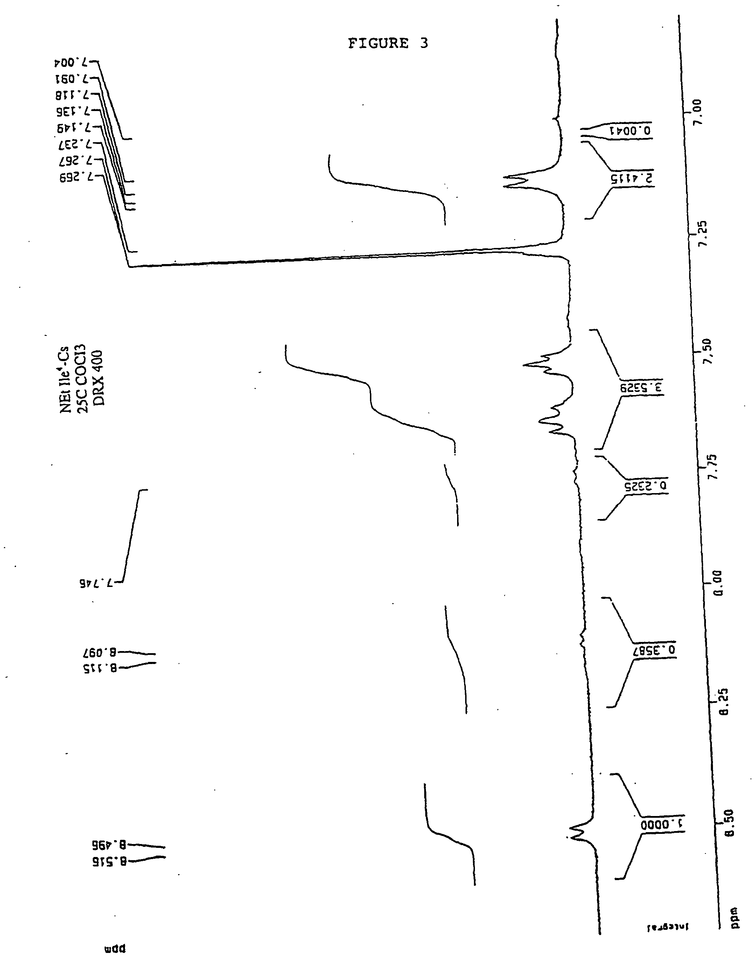

La figure 1 représente la structure générale de cette nouvelle cyclosporine. Les groupements R1, R2, R3 et R4 seront largement décrits à la Table III. Ainsi en transformant ces 4 positions clés il a été possible de conserver une très bonne affinité à la cyclophiline, et d'empêcher la formation d'un complexe ternaire avec la CaN et surtout d'augmenter de manière particulièrement avantageuse sa stabilité à l'oxydation enzymatique et par conséquent son activité anti-HIV.

Cette nouvelle cyclosporine est donc principalement caractérisée par la présence en position 4 d'un résidu avec R > CH₃ et R < -C₁₀ H₂₁. On utilisera comme substituant de l'azote par exemple l'éthyle, le propyle, le butyle ou le pentyle, mais ces exemples ne sont pas limitatifs. Cette nouvelle cyclosporine est particulièrement active lorsque le résidu en position 4 est un acide aminé N-éthylé (voir dessins 2 et 3).

L'invention revendique également la composition pharmaceutique de la substance telle que décrite par la formule (I). Celle-ci peut-être associée à une solution acceptable d'un point de vue pharmaceutique. La formule galénique ainsi produite permet d' augmenter la solubilité dans l'eau ou de maintenir la compostion sous forme de microémulsions en suspension dans l'eau. Le but de cette invention est également de fournir un nouveau médicament qui peut être utilisé par exemple dans le traitement et la prévention du SIDA (syndrome d'immunodéficience acquise). On utilisera tout particulièrement la cyclosporine modifiée en position 4 par un résidu Z étant N-éthyle-Valine pour la fabrication d'un médicament destiné au traitement et à la prévention du SIDA. L'application pour la prévention du SIDA n'est pas limitative. Cette substance peut aussi être employée par exemple pour ses propriétés anti-inflammatoires.

En ce qui concerne le procédé de fabrication de cette nouvelle cyclosporine nous avons utilisé les techniques classiques décrites dans la littérature ainsi que certaines méthodes spécifiques développées en laboratoire.

Le procédé de synthèse de la CsA est décrit dans : R.Wenger (Helv. Chim. Acta Vol 67 p. 502-525 (1984)). Le procédé d'ouverture de la cyclosporine A protégée (OAc) est décrit dans Peptides 1996. La molécule de CsA est traitée par le réactif de Meerwein (CH₃)₃OBF₄ puis scindée par traitement à l'acide dans le méthanol ou hydrolysée par l'eau, afin de la transformer en un peptide linéaire de 11 résidus d'acides aminés: H-MeLeu-Val-Meleu-Ala-(D)Ala-MeLeu-MeLeu-MeVal-MeBmt(OAc)-Abu-Sar-OCH3. Ce procédé a été présenté à la conférence internationale de la société européenne des Peptides (EPS-24) à Edimbourg 8-13 septembre 1996 et publié dans PEPTIDES 1996 par R.Wenger . Ce peptide linéaire est ensuite traité selon le procédé classique de Edman afin de scinder son dernier résidu d'acide aminé (MeLeu) et de fournir notre produit de départ: le décapeptide H-Val-MeLeu-Ala-(D)Ala-Meleu-MeVal-MeBmt(OAc)-Abu-Sar-OMe. Ce produit est ensuite utilisé dans les étapes suivantes:

### Préparation de (1) (protection):

### Boc-Val-MeLeu-Ala-(D) Ala-MeLeu-MeLeu-MeValMeBmt(OAc) -Abu-Sar-OMe (1)

A une solution de 2.83 g (2.46 mmoles) du décapeptide H-Val-MeLeu-Ala-(D)Ala-MeLeu-MeVal-MeBmt(OAc)-Abu-Sar-Ome dans 120 ml de dioxane sont ajoutés 0,72 ml (4.18 mmoles) d'une solution de diisopropyléthylamine et 0.65 g (2.95 mmoles) de Boc anhydride dans 50 ml de dioxane. On rajoute 17 ml d'eau à la solution qui est mélangée pendant 2 heures à température ambiante. Le solvant est alors évaporé et le mélange réactif résultant est dissous dans 300 ml d'acétate d'éthyle puis lavé 3 x avec une solution à 5 % d'acide citrique, 3 x avec une solution saturée en NaHCO₃ et enfin 3 x avec une solution en NaCl. Les phases organiques sont séchées au moyen de Na₂ SO₄ anhydre, filtrées et le solvant est finalement évaporé sous vide. On obtient ainsi 3 g (98%) du décapeptide protégé (Boc-décapeptide-méthyle ester).

Le produit est ensuite utilisé pour les voies de synthèse suivantes sans étape de purification supplémentaire. Cette substance est hydrolysée puis activée et condensée avec 1 acide aminé correspondant afin de produire un nouveau peptide à 11 résidus, produit de départ pour la cyclisation et la production d'une nouvelle cyclosporine aux propriétés désirées.

### Préparation de (2) (Hydrolyse de l'ester):

### Boc-Val-MeLeu-Ala-(D) Ala-MeLeu-MeLeu-MeValMeBmt(OAc) -Abu-Sar-OH (2)

A 4.08 g (3.26 mmoles) du composé précédent (I) dans 146 ml de tétrahydrofurane sont rajoutés goutte-à-goutte (à 15 °C) 192 mg (4.56 mmoles) de LiOH/H₂O dissous dans 36 ml d'eau. On agite le tout à 15°C. La réaction est complète au bout de 120 heures après l'addition successive de 5 portions respectivement de 1.4 équivalents de LiOH/H₂O chacunes. La solution obtenue est neutralisée par 0.1 N HCl et le solvant est ensuite évaporé. Le produit solide récupéré est alors dissout dans 500 ml d'acétate d'éthyle et lavé 2 x avec une solution à 5 % d'acide citrique et 2 x avec une solution de saumure. Les phases aqueuses sont extraites 4 x par l'intermédiaire de 50 ml d'acétate d'éthyle et les phases organiques regroupées sont alors séchées avec du Na₂SO₄ anhydre, filtrées et évaporées. On obtient ainsi 3.84 g (95 %) du composé (2). Le produit est alors utilisé sans purifications supplémentaires.

### Préparation de (3) (addition d'un nouvel acide aminé):

### Boc-Val-MeLeu-Ala-(D)Ala-Meleu-Meleu-MeVal-MeBmt(OAc)-Abu-Sar-EtVal-OtBu (3)

6.18 g (5 mmoles) du composé (2) sont dissous dans 250 ml de dichlorométhane anhydre sous argon. La solution est alors refroidie et on ajoute lentement sous argon 3.9 ml de N-méthylmorpholine (10 mmoles;pH 8.5) et 1.1 ml (10 mmoles) d'isobutylchloroformiate. La solution est agitée durant 15 minutes à -15°C. On ajoute alors pendant 20 minutes une solution de 2.4 g (12 mmoles) de H-NEt Val-OtBu dissous dans 40 ml de dichlorométhane anhydre. Le mélange est alors agité 1 heure à - 15°C, puis 1 heures à 0° C et enfin toute la nuit à température ambiante. Par la suite, on rajoute 400 ml de dichloromèthane puis on opère à 3 extractions par une solution d'acide citrique à 5 % suivie de 3 extractions par une solution saturée en NaHCO₃ et enfin 3 dernières extractions avec une solution saturée en NaCl. Les phases organiques sont séchées avec du Na₂SO₄ anhydre puis filtrées et finalement le solvant est évaporé. On récupère après chromatographie 4.42 g (62 %) de undécapeptide pur.

### Préparation de (4) (déprotection):

### H-Val-MeLeu-Ala-(D)-Ala-MeLeu-MeLeu-MeVal-MeBmt(OAc)-Abu-Sar-EtVal-OH (4)

830 mg (0.58 mmole) de undécapeptide protégé (4) sont dissous dans 15 ml de dichlorométhane pur. A cette solution on ajoute pendant 3 min. à température ambiante 3.2 ml d'acide trifluoroacétique. La réaction est suivie par une HPLC qui s'avère complète au bout de 1 h 30. Le solvant est évaporé et le restant de l'acide trifluoroacétique est évaporé 2 x en présence d'acétate d'éthyle.

Le produit brut (900 mg) est purifié par chromatographie [150 g de gel de silice (0.4-0.63)], utilisation comme éluants de dichlorométhane/méthanol/ triéthylamine ( 17:3:0.05 ) pour éluer 700 mg (95 %) de undécapeptide déprotégé (4) pur.

### Préparation de (5) (cyclisation):

### MeBmt(OAc)¹-EtVal⁴-Cs (5)

275 mg de TFFH (1.04 mmoles) sont dissous sous argon dans 3.45 1 de dichlorométhane anhydre. L'undécapeptide déprotégé (4) [438 mg (0.347 mmole)] est alors dissous dans 40 ml de dichlorométhane anhydre et 0.52 ml (3.82 mmoles) de collidine y sont ajoutés. Cette solution de peptide légèrement basique est ajoutée goutte-à-goutte à la solution de TFFH durant 20 min sous argon et agitation vigoureuse. Après 1 h 30 tout le matériel de départ est cyclisé. Pour emprisonner l'excès de TFFH on ajoute 5 ml d'eau puis la solution est évaporée. On ajoute 200 ml de dichlorométhane puis le tout est lavé respectivement 3 x avec une solution 0.1 N de HCl aqueux, 3 x avec une solution de saumure puis séché avec du Na₂SO₄, filtré et le solvant est évaporé. On obtient 360 mg d'une huile jaunâtre. Le produit brut est purifié par chromatographie en gel de silice en utilisant 100 g de gel de silice (0.04-0.0063 mm) et 1 % de méthanol dans de l'acétate d'éthyle comme éluant. On produit ainsi 230 mg (54 %) du dérivé (5) pur.

### Clivage du groupe acétate de MeBmt (OAc)-EtVal⁴-Cs (5) et production de EtVal⁴-Cs (6):

A une solution de 700 mg (0.562 mmole) du dérivé de Cs (5) dans 28 ml de Me OH on ajoute goutte-à-goutte sous argon 1.44 ml d'une solution 0.45 molaire de NaOCH₃ dans du MeOH (0.647 mmole). [La solution de NaOCH₃ dans le méthanol est préparée par addition de sodium au MeOH pur]. La réaction est complète après 48 h sous agitation à température ambiante. Le mélange est amené à pH 5 par addition de 50 % d'acide acétique dans l'eau. Les solvants sont éliminés sous vide. Le produit brut est dissous dans 200 ml d'acétate d'éthyle et extrait 2 x à l'eau. La phase aqueuse est réextraite avec 50 ml d'acétate d'éthyle puis les phases organiques combinées sont lavées 2x par une solution de saumure, séchées par du Na₂ SO₄ , filtrées et le solvant est évaporé.

Le produit obtenu (750 mg) est chromatographié sur 180 g de gel de silice (0.04-0.063 mm) en utilisant une solution d'acétone/hexane 1:1. (fractions de 20 ml). On produit ainsi 550 mg (82 %) de (EtVal⁴) Cs (6).

### Préparation de H-EtVal-Ot-Bu:

A une suspension de 5 g (23.8 mmoles) de H-ValOtBu x HCl dans 1 1 de triméthyloxoformiate, on ajoute sous argon 4.1 ml (23.83 mmoles) de diisopropyléthylamine. Au bout de 10 minutes la suspension devient claire. A cette solution on ajoute goutte-à-goutte en conditions anhydres 13.5 ml (0.24 mmole) d'acétaldéhyde dissous dans 30 ml de triméthyloxoformiate. La réaction est agitée pendant 45 minutes sous argon à température ambiante.

En utilisant un faible vide on élimine l'excès d'acétaldéhyde par évaporation durant 1 h 30. A cette solution, on ajoute sous argon 25 g (0.112 mmole) de NaBH(OAc)₃ solide. Après 15 minutes la solution est refroidie à 0°C et on ajoute lentement 500 ml d'une solution aqueuse d'HCl à 2 %.

Le triméthyloxoformiate est évaporé sous vide et le reste de la solution aqueuse est dilué dans 300 ml d'eau. Cette solution est alors extraite 2 x avec 100 ml de diéthyléther. La phase organique est ensuite réextraite 3 x avec une solution de HCl aqueux à 0.1 N. Les phases aqueuses recombinées sont refroidies à 0°C, puis le pH est amené à 9 au moyen de NaOH(2N). La solution devient alors trouble. La suspension aqueuse est extraite 4 x au moyen de 100 ml de diéthyléther. Les phases organiques recombinées sont ensuite séchées par le Na₂ SO₄ filtrées et le solvant est finalement évaporé.

4.2 g d'une huile jaunâtre résultant de cette étape sont purifiés par chromatographie en utilisant 900 g d'un gel de silice (0.04-0.063 mm) ainsi qu'un mélange d'hexane/acétate d'éthyle 8:2 comme éluant. On obtient finalement 3.13 g (65 %) de H-EtLeu-OtBu pur.

Les résultats de la table I montrent l'affinité des dérivés de Cs à la cyclophiline A dans un test ELISA compétitif décrit par Quesniaux dans Eur. J. Immunology 1987, 17, 1359-1365. Dans ce test lors de l'incubation avec la cyclophiline, on ajoute à la Cs à tester de la Cs liée à la BSA (sérum albumine). On calcule alors la concentration requise pour obtenir 50 % d'inhibition (CI₅₀) de la réaction témoin en absence de compétiteur. Les résultats sont exprimés par l'indice de liaison IL qui est le rapport de la CI₅₀ du dérivé et de la CI₅₀ de la CsA. Un indice de liaison (IL) de 1.0 indique que le composé testé se lie aussi bien que la CsA. Une valeur inférieure à 1.0 indique que le dérivé se lie mieux que la CsA, de même une valeur supérieure à 1.0 signifie que le dérivé se lie moins bien à la CyP que la CsA

**TABLE I**

| Substance | Structure | IL | IR |
|---|---|---|---|
| UNIL 001 | CsA | 1.0 | 1.0 |
| UNIL 002 | MeVal⁴-Cs | 0.6 | >200 |
| UNIL 004 | EtVal⁴-Cs | 1.0 | >200 |
| UNIL 007 | MeIle⁴-Cs | 0.5 | >200 |
| UNIL 013 | Et ILe4-Cs | 1.3 | > 200 |
| UNIL 014 | Et Phe(4-CH₂PO(OMe)₂)-Cs | 0.5 | >200 |

La Cs est considérée être immunosuppresive lorsque son activité dans la réaction de mélange lymphocytaire (MLR) est supérieure à 5 %. La réaction (MLR) est décrite par T. Meo dans "Immunological methods", L. Lefkovits et B. Devis, Eds, Académie Prev. N.Y. pp: 227-239 (1979).

Des cellules de la rate (0.5.10⁶) provenant de souris Balb/c (femelles, 8 à 10 semaines) sont co-incubées 5 jours en présence de cellules de la rate traitées provenant de souris CBA (femelles, 8 à 10 semaines). Ces cellules ont été traitées par la mitomycine C ou ont été irradiées à 2000 rads. Les cellules allogéniques de rate non irradiées présentent une réponse proliférative chez les cellules Balb/c que l'on peut mesurer par l'incorporation dans l'ADN d'un précurseur marqué. Lorsque les cellules stimulatrices sont irradiées (ou traitées à la mitomycine C) les cellules de Balb/c ne présentent plus une réponse proliférative mais conservent cependant leur antigénicité. La CI₅₀ calculée dans le test de MLR est comparée à la CI₅₀ correspondant à la CsA dans une expérience parallèle. On trouve ainsi l'indice IR étant le rapport de la CI 50 du test MLR des dérivés sur la CI₅₀ de la cyclosporine A.

De la même façon que pour l'indice de liaison (IL) précédent, une valeur de 1.0 pour l'IR signifie une activité similaire à la CsA. De même, une valeur inférieure signifie une meilleure activité et une valeur supérieure à 1.0 démontre une activité du composé inférieure à celle de la CsA.

Une valeur d'IR > 20 montre que la subtance est non-immunosuppressive. Les valeurs d'immunosuppression des dérivés sont données dans la table I.

La table II décrit le pourcentage de protection lors d'une infection par le HIV d'une lignée cellulaire CEM-SS. La protection de cette lignée en présence d'un dérivé de Cs est comparée à l'infection d'une lignée cultivée en absence de dérivé de Cs (témoin contrôle). Une valeur moyenne est établie à une concentration du dérivé de 2.10⁻⁶ molaire. La mesure de cette activité anti-HIV a été effectuée par le NCI (National Cancer Institute) à Washington aux USA.

**TABLE II**

| **Subtance** | **Structure** | **Pourcentage de protection à HIV** |
|---|---|---|
| UNIL 002 | MeVal⁴-Cs | 66.4 |
| UNIL 004 | EtVal⁴-Cs | 74.9 |
| UNIL 007 | MeIle⁴-Cs | 68.5 |

Un meilleur pourcentage de protection à l'infection par le HIV obtenu pour le composé Et Val⁴-Cs (comparé aux deux autres références connues pour être 10 x meilleures que la CsA) démontre l'avantage de la substitution par un N-éthyle en position 4. Cette remarque est encore plus valable lorsque l'on compare l'affinité à la CyP de chaque substance. On obtient pour le dérivé Et Val⁴-Cs une affinité à la Cyp semblable à celle de la CsA (IL=1.0) alors que les dérivés MeVal⁴-Cs et MeIle⁴-Cs démontrent une affinité à la Cyp supérieure (IL=0.6 et 0.5 respectivement). A une affinité à la Cyp plus faible de EtVal⁴-Cs correspondant une activité anti-HIV plus forte. Ceci démontre bien la valeur de cette nouvelle dérivation.

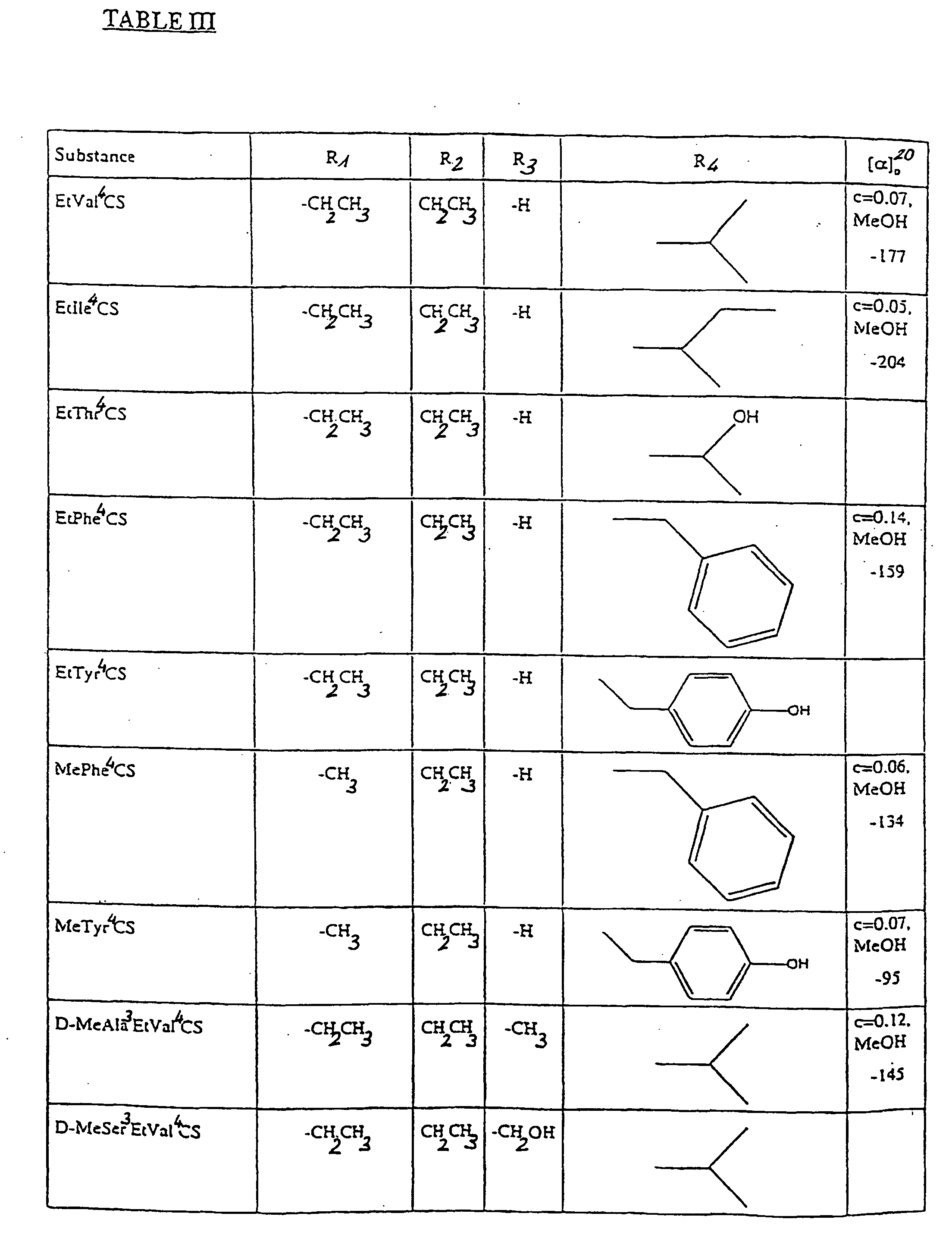

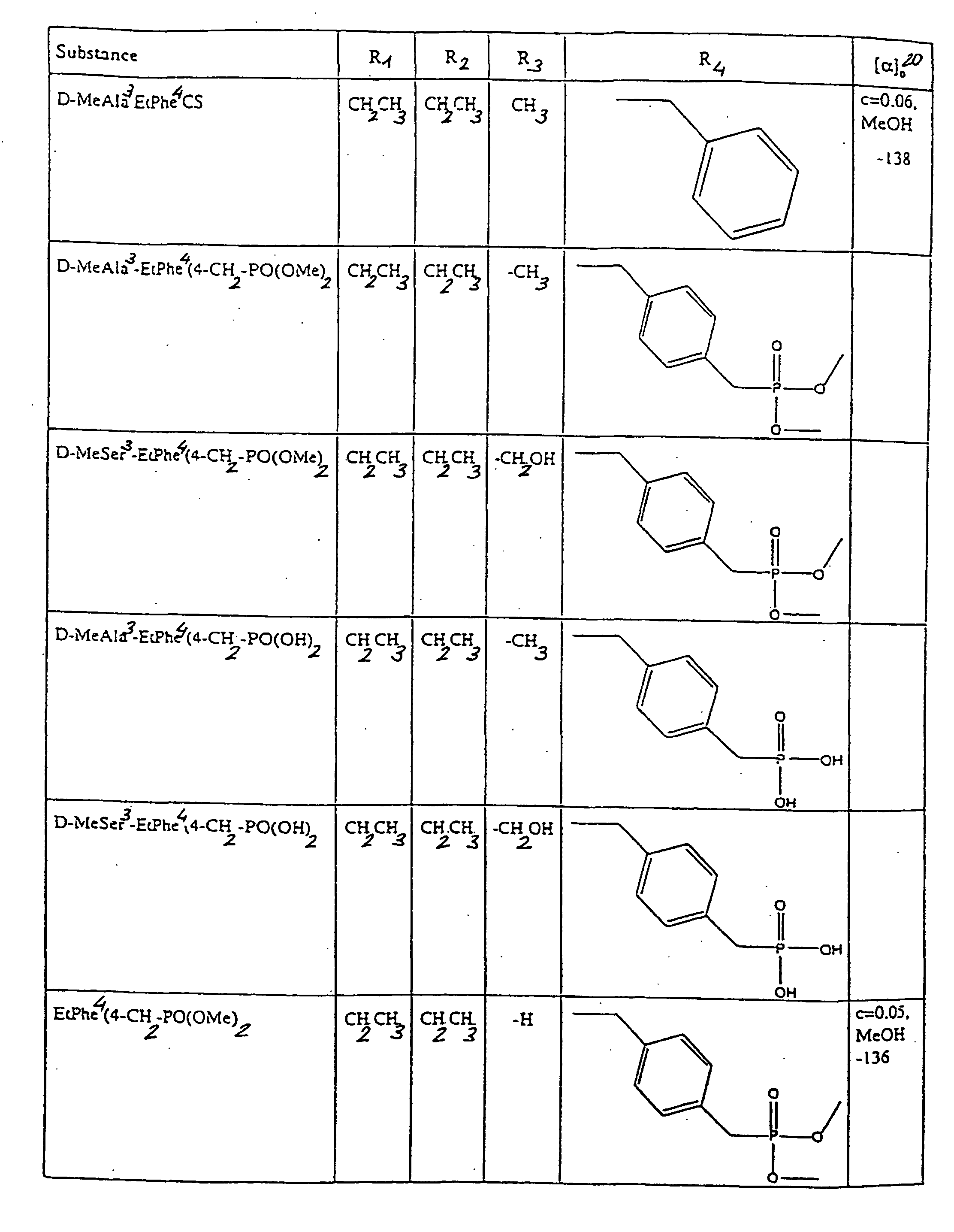

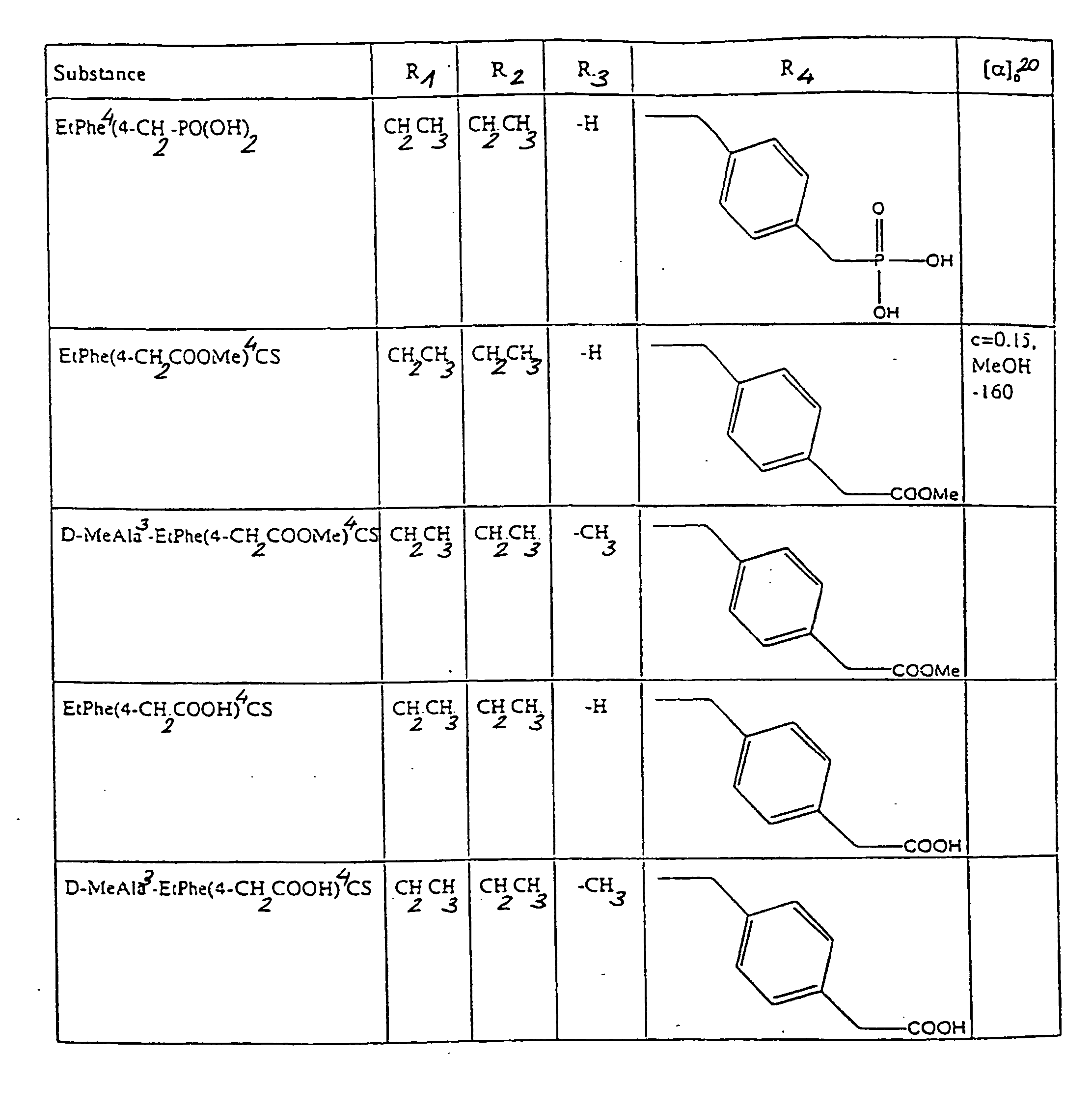

## Revendications

1. Cyclosporine de synthèse **caractérisée par** la formule: 
X—U—Y—Z—Val—MeLeu—Ala—(D) Ala—MeLeu—MeLeu—MeVal
1 2 3 4 5 6 7 8 9 10 11
 dans laquelle:
X est -MeBmt ou 6,7 dihydro-MeBmt-
U est-Abu, Nva, Val, Thr
Y est Sar ou (D)-MeSer ou (D)-MeAla ou(D)-MeSer (OAcyl)
Z est (N-R)aa où aa={Val, Ile, Thr, Phe, Tyr, Thr (OAc), Thr (OG₁),
Phe (G₂), PheCH₂(G₃), Tyr (OG₃)} avec R={alkyl > CH₃ et alkyl < -C₁₀H₂₁};
G₁= {Phényile-COOH, Phényle-COOMe, Phényle-COOEt};
G₂={CH₂COOH, CH₂COOMe, CH₂COOEt, CH₂PO(OMe)₂, CH₂PO(OH)₂};
G₃{PO(OH)₂,PO(OCH₂CH=CH₂)₂,CH₂COOH,CH₂COOMe, CH₂COOEt}.

2. Cyclosporine selon la revendication 1, **caractérisée en ce que** le résidu Z en position 4 est (N-R) Val.

3. Cyclosporine selon l'une quelconque des revendications précédentes **caractérisée en ce que** le résidu Z en position 4 est N-éthyle-Valine.

4. Composition pharmaceutique contenant le composé **caractérisé par** la formule: 
X—U—Y—Z—Val—MeLeu—Ala—(D) Ala—MeLeu—MeLeu—MeVal
1 2 3 4 5 6 7 8 9 10 11
 dans laquelle:
X est -MeBmt ou 6,7 dihydro-MeBmt-
U est -Abu, Nva, Val, Thr
Y est Sar ou (D)-MeSer ou (D)-MeAla ou(D)-MeSer (OAcyl)
Z est (N-R)aa où aa={Val, Ile, Thr, Phe, Tyr, Thr (OAc), Thr (OG₁),
Phe (G₂), PheCH₂(G₃), Tyr (OG₃)} avec R={alkyl > CH₃, et alkyl < -C₁₀H₂₁};
G₁={Phényle-COOH, Phényle-COOMe, Phényle-OOEt};
G₂={CH₂COOH, CH₂COOMe, CH₂COOEt; CH₂PO(OMe)₂, CH₂PO(OH)₂};
G₃ {PO(OH)₂, PO(OCH₂CH=CH₂)₂,CHCOOH, CH₂COOMe, CH₂COOEt}.

5. Composition pharmaceutique selon la revendication 4, **caractérisée en ce qu'**elle est associée à une solution acceptable au point de vue pharmaceutique.

6. Utilisation de la cyclosporine selon l'une quelconque des revendications précédentes pour la fabrication d'un médicament destiné au traitement et à la prévention du SIDA.

7. Utilisation de la cyclosporine selon la revendication 3 pour la fabrication d'un médicament destiné au traitement et à la prévention du SIDA.

## Patentansprüche

1. Synthetisches Cyclosporin, **gekennzeichnet durch** die Formel: 
X—U—Y—Z—Val—MeLeu—Ala—(D)Ala—MeLeu—MeLeu—MeVal
1 2 3 4 5 6 7 8 9 10 11
 worin:
X -MeBmt oder 6,7-Dihydro-MeBmt- ist,
u -Abu, Nva, Val, Thr ist,
Y Sar oder (D) -MeSer oder (D)-MeAla oder (D) -MeSer (OAcyl) ist,
Z (N-R)aa ist, wobei aa = {Val, Ile, Thr, Phe, Tyr, Thr (OAc), Thr (OG₁), Phe (G₂), PheCH₂(G₃), Tyr (OG₃)} mit R = {Alkyl > CH₃ und Alkyl < -C₁₀H₂₁}, G₁ = {Phenyl-COOH, Phenyl-COOMe, Phenyl-COOEt}, G₂ = {CH₂COOH, CH₂COOMe, CH₂COOEt, CH₂PO(OMe)₂, CH₂PO(OH)₂}, G₃ = PO(OH)₂, PO(OCH₂CH=CH₂)₂, CH₂COOH, CH₂COOMe, CH₂COOEt} ist.

2. Cyclosporin gemäß Anspruch 1, **dadurch gekennzeichnet, daß** der Rest Z an Position 4 (N-R)Val ist.

3. Cyclosporin gemäß einem der vorhergehenden Ansprüche, **dadurch gekennzeichnet, daß** der Rest Z an Position 4 N-Ethyl-Valin ist.

4. Pharmazeutische Zusammensetzung enthaltend die Verbindung, die durch die Formel **gekennzeichnet** ist: 
X—U—Y—Z—Val—MeLeu—Ala—(D)Ala—MeLeu—MeLeu—MeVal
1 2 3 4 5 6 7 8 9 10 11
 worin:
X -MeBmt oder 6,7-Dihydro-MeBmt- ist,
u -Abu, Nva, Val, Thr ist,
Y Sar oder (D)-MeSer oder (D)-MeAla oder (D)-MeSer (OAcyl) ist,
Z (N-R)aa ist, wobei aa = {Val, Ile, Thr, Phe, Tyr, Thr (OAc), Thr (OG₁), Phe (G₂), PheCH₂(G₃), Tyr (OG₃)} mit R = {Alkyl > CH₃ und Alkyl < -C₁₀H₂₁}, G₁ = {Phenyl-COOH, Phenyl-COOMe, Phenyl-COOEt}, G₂ = {CH₂COOH, CH₂COOMe, CH₂COOEt, CH₂PO(OMe)₂, CH₂PO(OH)₂}, G₃ = PO(OH)2, PO(OCH₂CH=CH₂)₂, CH₂COOH, CH₂COOMe, CH₂COOEt} ist.

5. Pharmazeutische Zusammensetzung gemäß Anspruch 4, **dadurch gekennzeichnet, daß** sie in einer pharmazeutisch annehmbaren Lösung vorliegt.

6. Verwendung von Cyclosporin gemäß einem der vorhergehenden Ansprüche zur Herstellung eines Medikaments zur Behandlung und Verhinderung von Aids.

7. Verwendung von Cyclosporin gemäß Anspruch 3 zur Herstellung eines Medikaments zur Behandlung und Verhinderung von Aids.

## Claims

1. Synthesised cyclosporin having the formula: 
X—U—Y—Z—Val—MeLeu—Ala—(D) Ala—MeLeu—MeLeu—MeVal
1 2 3 4 5 6 7 8 9 10 11
 wherein:
X is -MeBmt or 6,7-dihydro-MeBmt-
U is -Abu, Nva, Val, Thr
Y is Sar or (D)-MeSer or (D)-MeAla or (D)-MeSer (OAcyl)
Z is (N-R)aa wherein aa={Val, Ile, Thr, Phe, Tyr, Thr (OAc), Thr (OG₁), Phe (G₂), PheCH₂(G₃), Tyr (OG₃)} with R={alkyl>CH₃ and akyl<-C₁₀H₂₁};
G₁={phenyl-COOH, phenyl-COOMe, phenyl-COOEt};
G₂={CH₂COOH, CH₂COOMe, CH₂COOEt, CH₂PO(OMe)₂, CH₂PO(OH)₂} ;
G₃={PO(OH)₂, PO(OCH₂CH=CH₂)₂, CH₂COOH, CH₂COOMe, CH₂COOEt}.

2. Cyclosporin according to claim 1, **characterized in that** the residue Z in position 4 is (N-R)Val.

3. Cyclosporin according to any one of the preceding claims, **characterized in that** the residue Z in position 4 is N-ethyl-Valine.

4. Pharmaceutical composition containing the compound **characterized by** the formula: 
X—U—Y—Z—Val—MeLeu—Ala—(D) Ala—MeLeu—MeLeu—MeVal
1 2 3 4 5 6 7 8 9 10 11
 wherein:
X is -MeBmt or 6,7-dihydro-MeBmt-
U is -Abu, Nva, Val, Thr
Y is Sar or (D)-MeSer or (D)-MeAla or (D)-MeSer (OAcyl)
Z is (N-R)aa wherein aa={Val, Ile, Thr, Phe, Tyr, Thr (OAc), Thr (OG₁),
Phe (G₂), PheCH₂(G₃), Tyr (OG₃)} with R={alkyl>CH₃ and alkyl<-C₁₀H₂₁} ;
G₁={phenyl-COOH, phenyl-COOMe, phenyl-COOEt};
G₂={CH₂COOH, CH₂COOMe, CH₂COOEt, CH₂PO(OMe)₂, CH₂PO(OH)₂};
G₃= {PO(OH)₂, PO(OCH₂CH=CH₂)₂, CH₂COOH, CH₂COOMe, CH₂COOEt}.

5. Pharmaceutical composition according to claim 4, **characterized in that** it is combined with a pharmaceutically acceptable solution.

6. Use of the cyclosporine according to any one of the preceding claims for the manufacture of a medicament for treating and preventing AIDS.

7. Use of the cyclosporine of claim 3 for the manufacture of a medicament for treating and preventing AIDS.
